# FASCICULE DE BREVET EUROPEEN

(11) **EP 2 120 772 B1**
(45) Date de publication et mention de la délivrance du brevet: **17.07.2013**
(21) Numéro de dépôt: 07872027.3
(22) Date de dépôt: 24.12.2007
(51) Int. Cl.: A61C 1/08, A61B 17/16

(54) **DISPOSITIF DE GUIDAGE ET DE MODELAGE OSSEUX POUR LA PRÉPARATION DE SITES OSSEUX EN CHIRURGIE IMPLANTAIRE**
KNOCHENMODELLIERUNG UND FÜHRUNGSVORRICHTUNG ZUR VORBEREITUNG VON KNOCHENSTELLEN ZUR IMPLANTATSOPERATION
BONE MODELLING AND GUIDE DEVICE FOR PREPARING BONE SITES FOR IMPLANT SURGERY

(30) Priorité: 02.01.2007 FR 0700011
(43) Date de publication de la demande: 25.11.2009
(73) Titulaire: Isidori, Michel, 69300 Caluire Et Cuire (FR)
(72) Inventeur: Isidori, Michel, 69300 Caluire Et Cuire (FR)
(74) Mandataire: Dupuis, François
(86) Numéro de dépôt international: PCT/FR2007/052618
(87) Numéro de publication internationale: WO 2008/087356

(56) Documents cités:
- WO-A-98/48707
- FR-A- 2 878 429
- FR-A- 2 882 250

## Description

La présente invention décrit :
1. Un dispositif de guidage axial pour la préparation de sites osseux en chirurgie générale ou dentaire, dans le but de réduire la dispersion angulaire ; ce dispositif amène une amélioration notable afin :
   - d'éviter la dégradation des tissus en chirurgie minimalement invasive, en contrôlant la profondeur d'enfouissement,
   - d'évaser la gencive ou muqueuse avant le passage du foret terminal par un dessin particulier,
   - d'éviter la rotation du dispositif et donc les risques de fracture du guide chirurgical,
   - de préparer des sites osseux déficients de façon très précise, en évitant les déviations générées par les tables osseuses.
2. Les outils de préparation correspondants afin d'utiliser ce dispositif quel que soit le type de site à préparer; éclatement de crête, expansion osseuse, élévation sous sinusienne, prélèvement osseux, forage, pose d'implant.

Il est bien entendu que ces dispositifs et outils sont utilisables pour la mise en place d'implants en vue de restaurations partielles ou totales à l'aide de prothèse scellée ou vissée, que ce soit par chirurgie avec lambeau ou bien par chirurgie minimalement invasive.

### Arrière plan technologique de l'invention

A l'heure actuelle, en chirurgie dentaire, la perte d'une ou plusieurs dents manquantes maxillaires ou mandibulaires est vécue bien souvent comme un drame et la mise en place d'une prothèse amovible, afin de compenser ces pertes dentaires, ne doit pas être considérée comme la meilleure solution.

Des solutions fixes, à l'aide de prothèses ancrées sur un ou plusieurs implants, peuvent être proposées. Les implants sont vissés ou impactés dans les sites osseux préparés de différentes façons :
- La technique la plus ancienne nécessitait une chirurgie osseuse « large » où les sites osseux étaient largement exposés (en décollant les gencives) afin de visualiser l'anatomie complexe du maxillaire supérieur ou de la mandibule, puis les sites osseux étant préparés à l'aide de forets de diamètre croissant de manière homothétique aux implants que l'on souhaitait poser, enfin les tissus gingivaux étaient remis en place, suturés et une phase de cicatrisation commençait pour 4 à 6 mois.
- Les techniques modernes d'imagerie médicale couplées à des techniques de robotique permettent, depuis quelques années, en association avec le guide radiologique testé en situation à appui dentaire ou muqueux :
   * de planifier sur ordinateur la position précise de nos implants en fonction de la réalisation prothétique finale validée préalablement en bouche,
   * de transférer ces données à un robot permettant de percer le guide radiologique et les modèles d'étude selon les axes de planification et donc,
   * de transférer la planification de l'ordinateur directement sur le site chirurgical permettant la réalisation de chirurgie précise et peu ou pas mutilante,
   * de positionner des implants de manière précise,
   * d'obtenir de bons résultats en diminuant les complications postopératoires.

Une des techniques actuellement utilisée, servant de base à l'invention, a été décrite dans le brevet FR 2.760.349 et le brevet US 6.296.483 B1. Parmi les autres techniques actuellement testées et validées, il y a lieu de se référer au gabarit chirurgical développé et décrit dans le brevet FR 2.836.372 *« gabarit à appui osseux réalisé par stéréolithographie ainsi que les techniques de préparation de sites osseux à l'aide des principes de navigation* ».

Dernièrement, un brevet décrivant la conception du guide implantaire ainsi qu'un dispositif de guidage a été déposé et publié sous le numéro FR 2.882.250 au nom du demandeur.

Par rapport aux inventions de l'art antérieur décrites précédemment, l'originalité de cette invention se situe dans la conception du dispositif de guidage afin de réaliser des chirurgies minimalement invasives en ne détériorant pas les tissus opérés, en contrôlant l'enfoncement du dispositif de guidage selon l'invention, donc en apportant encore plus de sécurité dans la profondeur de forage et plus de précision aux gestes chirurgicaux.

Actuellement, de nombreuses publications vantent les mérites d'une chirurgie assistée par ordinateur et les bienfaits d'une réalisation prothétique placée peu de temps post-opératoire, mais la précision apportée semble aléatoire, les piliers connectant la prothèse aux implants étant expansifs afin de compenser les erreurs d'axe.

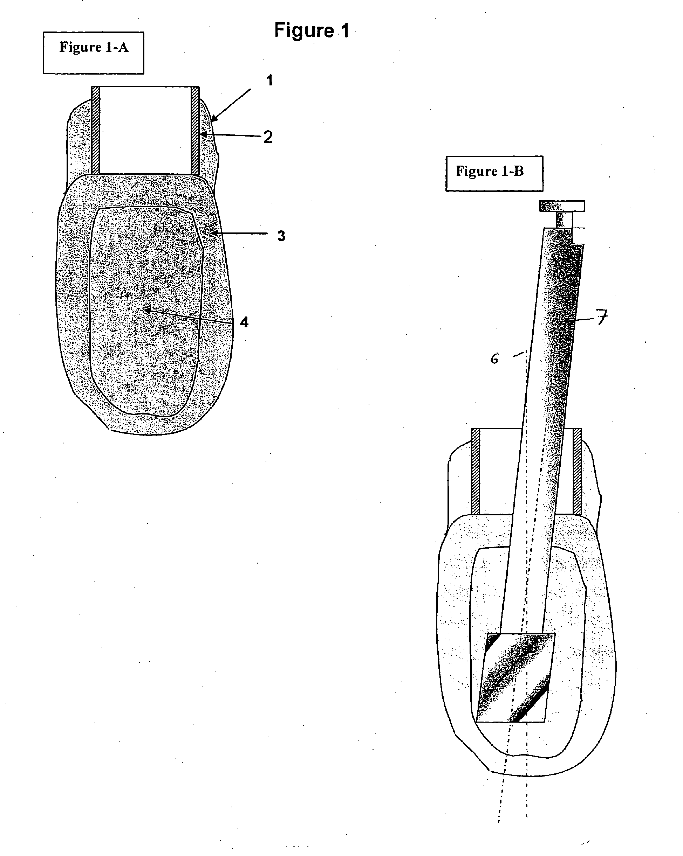

On a illustré aux figures 1 (1A - 1B), l'art antérieur précité.
**Figure 1** **:** A l'heure actuelle, les praticiens utilisent un guide chirurgical (1) placé sur la gencive (3) ou l'os (4) (après avoir enlevé des lambeaux). Ce guide est soit stabilisé par les dents résiduelles, soit par des vis latérales. En général, le guide a été perforé de manière manuelle, en fonction du positionnement des dents du montage directeur (wax up) ; certains praticiens réalisent des perforations de 2mm ou plus.
**Figure 1-A** **:** Dans la chirurgie assistée par ordinateur, à l'aide du système CaD-implant® , les perforations sont :
   * de diamètre 5,5 mm à travers le guide en résine dans lesquelles on insère des tubes (2) en acier chirurgical ou titane dans lequel on insère des tubes de diamètre croissant afin de réaliser les puits osseux ou :
   * de diamètre 5,0 mm dans lequel on place des tubes gigognes de diamètre croissant afin de réaliser les puits osseux.

**Sur la** **figure 1-B****,** on remarque que, lors du forage, une certaine déviation d'axe est possible, surtout lorsqu'on n'utilise pas des tubes gigognes. Leur utilisation apporte une amélioration notable de l'axe réalisé, par rapport à l'axe souhaité mais, du fait de la tolérance mécanique nécessaire entre deux pièces qui coulissent, une certaine source d'erreur existe. Il y a une différence entre l'axe prévu lors de la programmation (6) et l'axe obtenu (7), inacceptable dans une « mise en charge immédiate ».

Afin de réaliser des perçages précis pour placer rapidement la prothèse préparée, il est évident que la technique de forage osseux doit être améliorée. Plusieurs auteurs ont décrit différentes techniques de guidage de forets à l'aide de tubes (gigognes ou non), de diamètre croissant en fonction du diamètre croissant des forets mais avec les problèmes de tolérance mécanique, la précision souhaitée, pour mettre en place une prothèse préparée à l'avance, ne peut être que difficilement atteinte.

Les présentes inventions, selon l'art antérieur, concernent un dispositif universel de guidage axial de foret à utiliser lors de la préparation de sites osseux en chirurgie générale ou dentaire dans le but de réduire la dispersion angulaire, de contrôler la profondeur du forage et de respecter les tissus, ce dispositif pouvant toujours travailler avec les outils de coupe (forets, trépans) décrits dans la demande de brevet FR 2.882.250.

Le dispositif de guidage proposé dans le brevet FR 2.882.250 a permis de remédier aux problèmes de déviation d'axe. Il se présente sous la forme d'un cylindre que l'on enchâsse dans les guides chirurgicaux avec un guidage axial du foret, en contraignant ce dernier à se mouvoir uniquement selon l'axe de l'alésage.

Cependant, il n'apporte qu'une précision relative quant à la profondeur de forage, le foret pouvant emmener axialement le dispositif de guidage jusqu'à la gencive, et même parfois jusqu'au rebord osseux, provoquant une détérioration de cette gencive. De plus, il est libre en rotation, provoquant parfois la fracture du guide chirurgical.

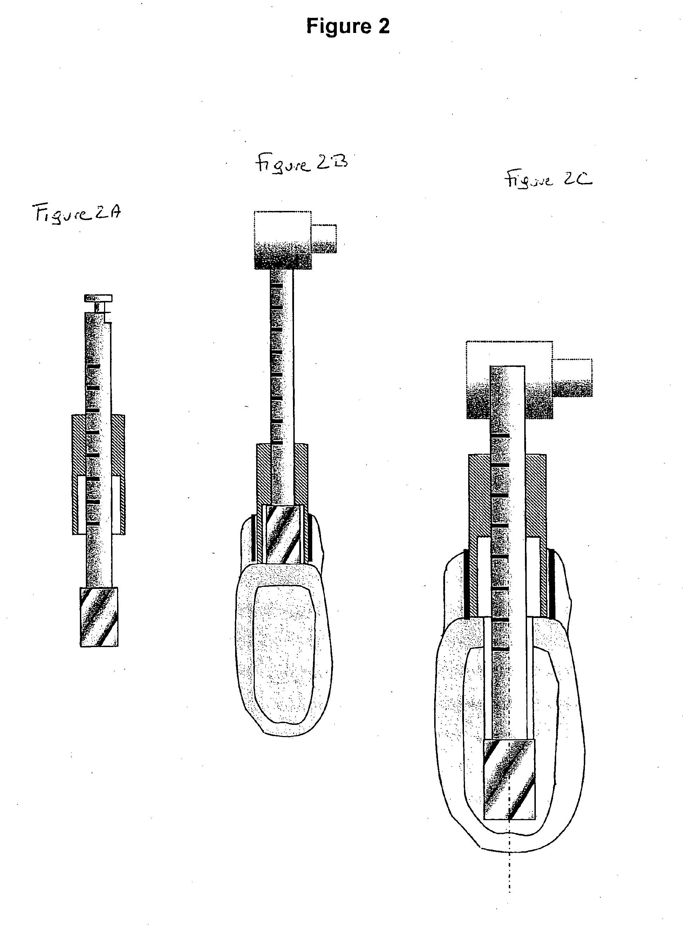

**La** **figure 2** (2A - 2B - 2C) représente la schématisation de l'utilisation du dispositif de guidage décrit dans le brevet FR 2.882.250 en association avec de nouveaux forets à développer selon l'invention. Le dispositif de guidage proposé permet un travail dans l'axe du foret mais n'a pas de blocage dans le sens vertical et peut s'enfoncer, provoquant une lésion des tissus par la rotation du dispositif de guidage. De plus, la profondeur du forage est assurée seulement par le contrôle visuel, sans sécurité. On a représenté, figure 2, le dispositif de guidage selon ce document.

WO 98/48707 A divalgue un dispositif de guidage selon le préambule de la revendication 1. FR 2878 429 divulgue un dispositif de forage pour l'insertion d'un implant dentaire comportant un bras de préhension.

### OBJET DE L'INVENTION

La présente invention vise à remédier aux insuffisances de l'art antérieur.

La présente invention concerne un dispositif de guidage et de modelage afin de préparer les sites osseux, et de nouveaux instruments de préparation osseuse chirurgicale et de pose d'implant permettant d'apporter au praticien une chirurgie sécurisée, peu stressante, valorisant son plan de traitement prothétique et au patient, une chirurgie précise, plus rapide, moins évasive, plus confortable, selon la revendication 1.
1. **Un dispositif de guidage axial de foret** lors de la préparation de sites osseux en chirurgie générale ou dentaire, dans le but de réduire la dispersion angulaire, de respecter les tissus opérés et de régler l'enfouissement de(s) l'implant(s), c'est-à-dire la profondeur, afin d'utiliser ce dispositif quel que soit le type de site à préparer dans le cadre de chirurgie planifiée par ordinateur.
2. Ce dispositif de guidage permettra également un guidage total lors de l'utilisation d'un porte implant guidé, des outils de modelage osseux (expanseur manuel ou mécanique, outil d'élévation sous sinusienne manuel ou mécanique communément appelé à tort « ostéotome », ciseaux à os, trépan ou tréphine osseuse).
3. Le dessin des nouveaux outils

Ainsi, selon une première caractéristique de l'invention, le dispositif de guidage et de modelage osseux est remarquable en ce qu'il comprend un corps cylindrique monobloc agencé et défini par deux portées cylindriques adjacentes et concentriques, l'une extérieure et l'autre intérieure, et en ce que la portée cylindrique extérieure étant établie avec au moins deux moyens d'indexation s'ajustant dans des formes complémentaires formées sur une bague solidaire d'un guide chirurgical, et en ce que la portée intérieure autorise le passage et le guidage de l'axe de l'outil de forage, et en ce que l'extrémité inférieure de la portée cylindrique externe est biseautée, et en ce que les moyens assurent le blocage du dispositif de guidage en profondeur et en rotation lors du positionnement du corps dans la bague encastrée dans le guide chirurgical.

Ces caractéristiques et d'autres encore ressortiront bien de la suite de la description.

Pour fixer l'objet de l'invention illustrée de manière non limitative aux figures des dessins où :
- La figure 1, et ses vues particulières 1A et 1B, sont des vues de l'art antérieur.
- La figure 2 est une vue de l'art antérieur correspondant au brevet FR 2.882.250.
- Les figures 3A, 3B illustrent le dispositif de guidage, selon l'invention, en phase initiale, puis en début de chirurgie.
- La figuré 4 illustre le dispositif de guidage de l'invention en phase de contact avec la muqueuse ou gencive.

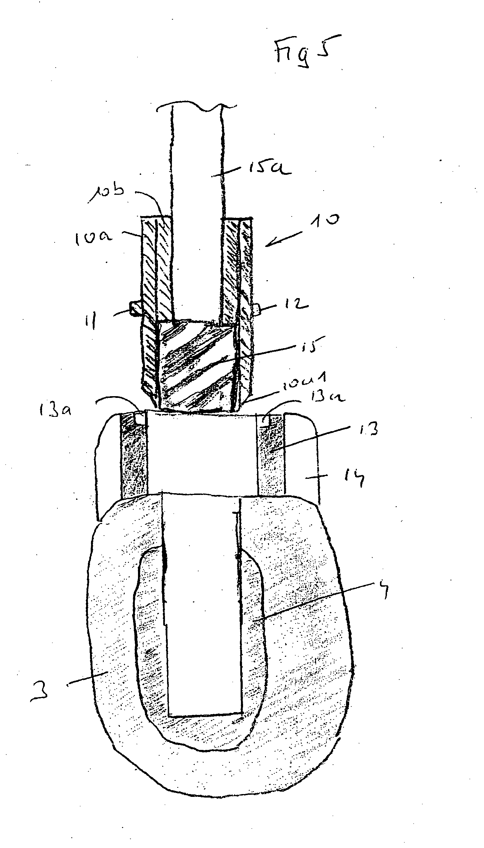

- La figure 5 est une vue partielle montrant l'extraction du dispositif.

Afin de rendre plus concret l'objet de l'invention, on le décrit maintenant d'une manière non limitative illustrée aux figures des dessins.

Le dispositif de guidage et de modelage, selon l'invention, référencé dans son ensemble par (UG) comprend un corps cylindrique (10) monobloc agencé et défini par deux portées cylindriques (10a - 10b) adjacentes et concentriques, l'une extérieure (10a), l'autre intérieure (10b). Ces deux portées cylindriques sont de hauteur différente ou égale. Ces deux portées sont de diamètre intérieur différent. La portée cylindrique extérieure (10a) est établie avec au moins deux moyens d'indexation (11 - 12) qui peuvent être des ergots, glissières ou taquets extérieurs, diamétralement opposés lorsqu'il y en a deux, et venant s'encastrer dans un nombre équivalent de formes complémentaires (13a) du type rainures ou encoches ou gorge périphérique formées sur une bague métallique (13) solidaire d'un guide chirurgical (14) bloquant ainsi le dispositif de guidage à la fois en rotation et en profondeur. L'extrémité inférieure de la portée cylindrique extérieure (10a1) est biseautée. La portée cylindrique intérieure est établie pour autoriser le passage et guidage de l'axe (15a) de l'outil récepteur de l'outil (15) lui-même constituant le foret.

Selon une caractéristique importante, la portée cylindrique (10a) définit deux zones (Z1 - Z2) successives en hauteur définies comme suit. La zone (Z1) est la partie libre sortante du corps après son positionnement sur la bague (13). La zone (Z2) est la partie intérieure se trouvant dans la bague après positionnement du corps dans la bague. La zone (Z2) est de hauteur constante de l'ordre de 5mm. La zone (Z1) est de hauteur variable de l'ordre de 3 à 20 mm permettant de contrôler la profondeur de forage et/ou de préparation de la gencive.

Ce dispositif médical, selon l'invention, peut être en acier chirurgical, en titane ou alliage de titane, en P.I.C. ou en POM (PolyOxyMéthacrylate), en céramique ou zircone et leurs oxydes, ou tout autre matériau utilisable dans les domaines biologiques ou/et médicaux.

Il est précisé que ce dispositif de guidage (UG) associé à sa bague (13) peut être utilisé avec tout guide chirurgical (14) perforé classiquement ou bien en insérant directement le dispositif de guidage (UG) dans le guide percé comportant au moins deux encoches diamétralement opposées deux à deux.

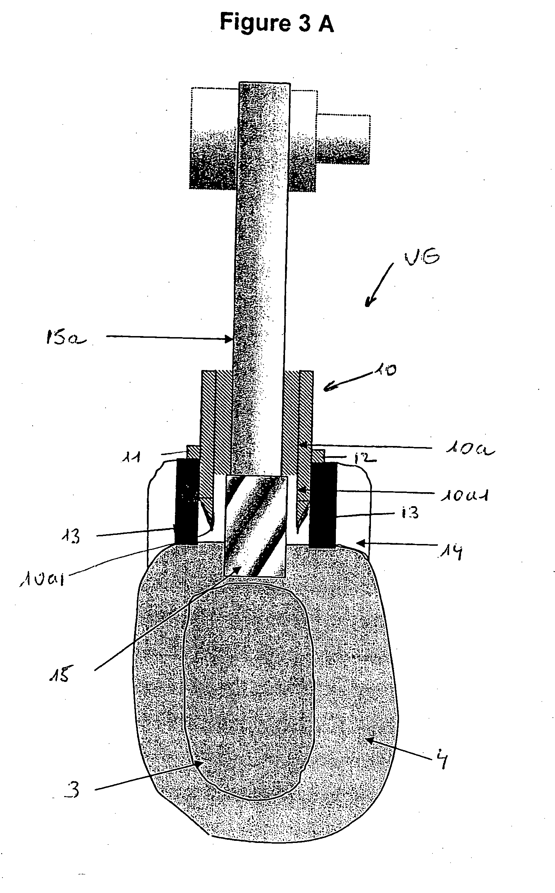

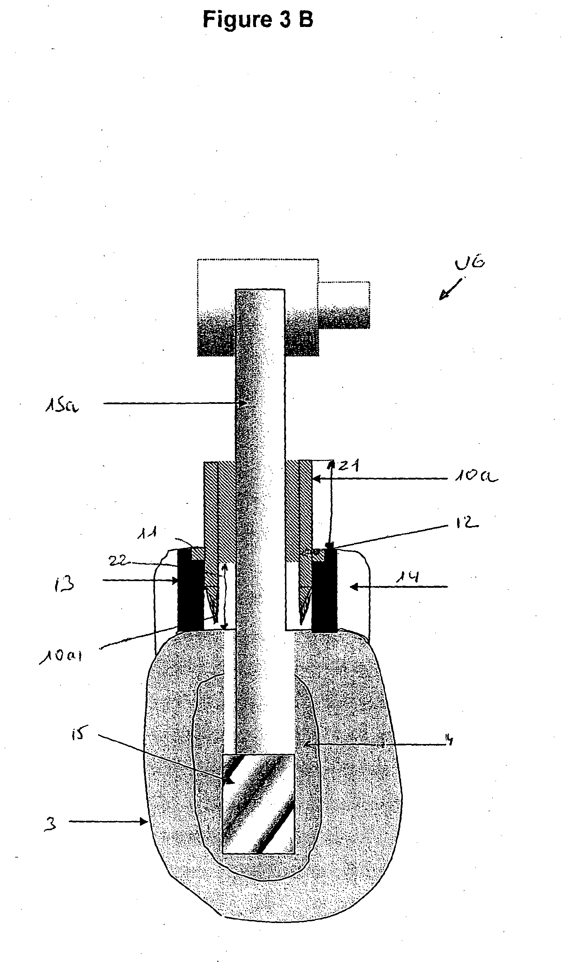

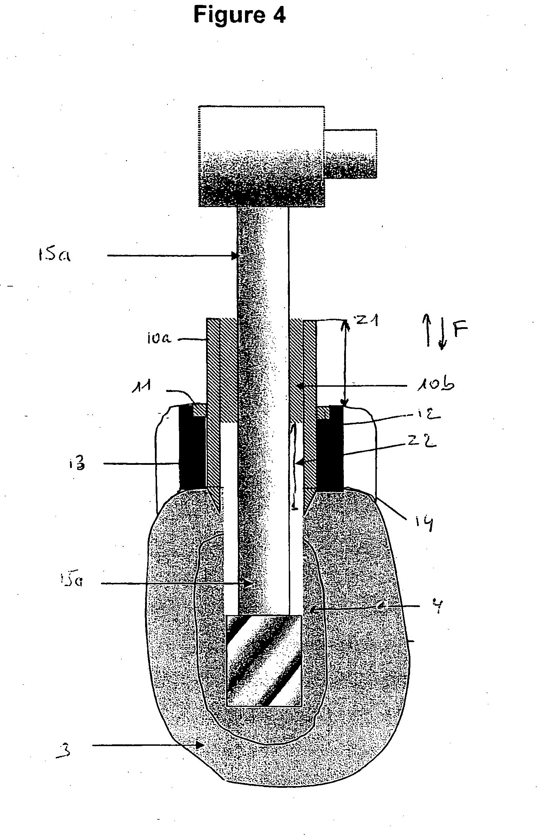

Il y a lieu de préciser que le mouvement de l'outil et du guide, représenté aux figures 3A, 3B, 4, est établi selon la flèche F dans un sens de va et vient vertical selon les besoins et que le retrait de l'outil entraîne l'enlèvement du corps de guidage de la gencive.

Le contrôle de la profondeur de forage se fait par la hauteur de cette zone Z1 : elle peut varier de 3,0mm à 15mm. Le diamètre maximal de la portée cylindrique intérieure (10b) de 4,60mm permet l'utilisation de tous les diamètres de foret ou outils nécessaires au cours des interventions chirurgicales de pose d'implant, la profondeur de cet alésage est au minimum de 5mm et peut varier en fonction de la hauteur de la partie travaillante du foret universel c'est-à-dire de 5mm à 10mm.

Le dispositif selon l'invention est destiné au guidage de tout outil servant à la préparation de site osseux en chirurgie ou implantologie, qu'il soit mécanique ou manuel ou pneumatique et des instruments de pose d'implant. L'extrémité (10a1) de la portée cylindrique (10a) du dispositif de guidage se termine en son extrémité par un biseau émoussé, afin de repousser les tissus, tel que représenté à la figure 4. Pour terminer la préparation du site implantaire et pour la pose d'implant, le dispositif de guidage selon l'invention sera choisi avec une hauteur de partie (Z1) plus élevée afin qu'il descende au contact gingival ou osseux en évasant la muqueuse.

Toutes ces explications rendent évidentes, pour le praticien, les avantages importants que le dispositif, selon l'invention, apporte à lui même et au patient en terme de précision, de sécurité, en terme de qualité et rapidité, en terme d'efficacité. Ce dispositif de guidage (UG) et sa bague peuvent bien évidemment être utilisés pour tout autre procédé ou protocole de chirurgie assistée par ordinateur, utilisé tel que décrit ou adapté à la technique employée.

On fait référence aux figures 3A, 3B et 4 pour des caractéristiques complémentaires de l'invention.

**Figure 3-A** **-** Le dispositif de guidage proposé selon l'invention peut coulisser librement le long de l'axe du foret ou outil de préparation osseuse avec une tolérance très faible ;
● L'axe de l'outil possède un marquage millimétrique permettant un premier contrôle de la profondeur de pénétration ;
● Le dispositif de guidage, présentant au moins deux ergots ou taquets s'encastrant dans la bague incluse dans le guide chirurgical, ne peut pas s'enfoncer et abîmer les tissus sous-jacents ;
● Le contrôle de la profondeur se fait par le choix de la hauteur du dispositif de guidage dans sa partie haute qui bloque la descente des instruments de travail en fin de course ; un deuxième contrôle s'effectue « de visu » par les repères millimétriques portés sur les queues des instruments de travail.

La **figure 3-B** présente le dispositif selon l'invention en situation réelle en début de chirurgie, le dispositif de guidage (10) coulissant le long de l'axe de l'outil du type foret (15), dans sa partie haute ou zone (Z1) en contrôlant en permanence la profondeur de pénétration de la partie travaillante du foret dans le site osseux par le blocage du dispositif de guidage dans la bague associée au guide chirurgical et la hauteur variable de la partie ou zone (Z1) comportant la portée cylindrique adaptée au diamètre de l'axe de l'outil de coupe ou de préparation.

La **figure 4** illustre le dispositif de guidage adapté au diamètre terminal de préparation du site osseux et pour la pose d'implant. Le dispositif de guidage selon l'invention sera choisi avec une hauteur de la portion de la zone Z2 plus élevée afin d'obtenir un contact dispositif de guidage - muqueuse ou gencive : le biseautage du dispositif de guidage permet de déjeter les tissus vers l'extérieur.

En conclusion, les avantages du dispositif selon l'invention sont :
1. Grande précision dans la technique chirurgicale,
2. Simplicité du dispositif de guidage,
3. Contrôle de la profondeur, respect des tissus, sécurité,
4. Prédictibilité, rapidité, traçabilité, peu ou pas de chirurgie,
5. Dispositif de guidage selon l'invention utilisable avec les instruments de coupe décrits dans le brevet FR 2.882.250,
6. Développement d'instrument, manuel ou mécanique, de préparation de sites osseux permettant un guidage sans déviation,
7. Dispositif de pose d'implant guidée évitant les déviations d'axe par rapport à une pose manuelle ou mécanique.

## Revendications

1. Dispositif de guidage et de modelage osseux (UG) comprenant un corps cylindrique monobloc (10) muni d'un alésage pour le guidage axial d'un foret (15), et une bague (13), l'extrémité inférieure dudit corps cylindrique monobloc (10) étant biseautée afin de déjeter les tissus vers l'extérieur, le corps cylindrique monobloc (10) comportant deux portées cylindriques (10a-10b) adjacentes et concentriques, une première portée dite intérieure (10b) autorisant le passage et guidage de l'axe (15a) de l'outil (15) de forage et une seconde portée dite extérieure (10a) comprenant au moins deux moyens d'indexation (11-12) s'ajustant dans des formes complémentaires (13a) formées sur ladite bague (13) apte à être solidarisée à un guide chirurgical (14), **caractérisé en ce que** les moyens d'indexation (11-12) assurent le blocage du dispositif de guidage en profondeur et en rotation lors du positionnement du corps cylindrique monobloc (10) dans la bague (13) solidarisée au guide chirurgical, et ladite portée intérieure (10b) de diamètre extérieur identique au diamètre intérieur de la portée extérieure (10a) et de diamètre intérieur différent du diamètre intérieur de la portée extérieure (10a), de telle sorte que la portée cylindrique extérieure (10a) définisse deux zones (Z1-Z2) successivement en hauteur, la zone (Z1) étant la partie libre sortante du corps cylindrique (10) après son positionnement sur la bague (13), la zone (Z2) étant la partie intérieure se trouvant dans la bague (13) après positionnement du corps cylindrique (10) dans la bague (13), ladite zone (Z1) étant de hauteur variable pour contrôler la profondeur de forage et/ou de préparation de la gencive, et la zone (Z2) étant de hauteur constante.

2. Dispositif selon la revendication 1, **caractérisé en ce que** les deux portées (10a)et (10b) sont de hauteur différente.

3. Dispositif selon la revendication 1, **caractérisé en ce que** les deux portées (10a)et (10b) sont de hauteur égale.

4. Dispositif selon la revendication 1, **caractérisé en ce que** la hauteur de la zone (Z1) est établie entre 3 et 15mm, et la hauteur constante de la zone (Z2) est établie à environ 5 mm.

5. Dispositif selon l'une quelconque des revendications 1 à 4, **caractérisé en ce que** les moyens d'indexation (11-12) consistent dans des ergots, glissières ou taquets extérieurs venant s'encastrer dans un nombre équivalent de formes complémentaires du type rainures ou encoches ou gorges périphériques formées sur la bague (13).

6. Dispositif selon la revendication 5, **caractérisé en ce que** les ergots, glissières ou taquets extérieurs sont diamétralement opposés.

7. Dispositif selon l'une quelconque des revendications 1 à 6, **caractérisé en ce qu**'il est obtenu dans de l'acier chirurgical, du titane ou un alliage de titane, dans du P.I.C. ou dans du P.O.M. (PolyOxyMéthacrylate), dans de la céramique ou du zircone et leurs oxydes.

8. Dispositif selon l'une quelconque des revendications 1 à 7, **caractérisé en ce que** le biseau formant l'extrémité (10a1) de la portée intérieure (10a) est émoussé.

## Patentansprüche

1. Vorrichtung zur Knochenführung und -modellierung (UG), die einen zylindrischen Monoblockkörpers (10), der mit einer Bohrung für die axiale Führung eines Bohrers (15) versehen ist, und einen Ring (13) umfasst, wobei das untere Ende des zylindrischen Monoblockkörpers (10) abgefast ist, um Gewebe nach außen zu biegen, wobei der zylindrische Monoblockkörper (10) zwei zylindrische Bereiche (10a-10b) besitzt, die zueinander benachbart und konzentrisch sind, wobei ein erster so genannter innerer Bereich (10b) den Durchgang und die Führung der Achse (15a) des Bohrwerkzeugs (15) zulässt und wobei ein zweiter so genannter äußerer Bereich (10a) wenigstens zwei Indexierungsmittel (11-12) enthält, die sich in komplementären Formen (13a) einstellen, die an dem Ring (13), der mit einer chirurgischen Führung (14) fest verbunden werden kann, gebildet sind, **dadurch gekennzeichnet, dass** die Indexierungsmittel (11-12) die Blockierung der Führungsvorrichtung in Tiefenrichtung und rotatorisch bei der Positionierung des Monoblockkörpers (10) in dem mit der chirurgischen Führung fest verbundenen Ring (13) sicherstellen und der innere Bereich (10b) einen Außendurchmesser besitzt, der mit dem Innendurchmesser des äußeren Bereichs (10a) übereinstimmt, und einen Innendurchmesser besitzt, der von dem Innendurchmesser des äußeren Bereichs (10a) verschieden ist, derart, dass der äußere zylindrische Bereich (10a) zwei in Höhenrichtung aufeinander folgende Zonen (Z1-Z2) definiert, wobei die Zone (Z1) der freie Teil ist, der aus dem zylindrischen Körper (10) austritt, nachdem er an dem Ring (13) positioniert worden ist, wobei die Zone (Z2) der innere Teil ist, der sich in dem Ring (13) befindet, nachdem der zylindrische Körper (10) in dem Ring (13) positioniert worden ist, wobei die Zone (Z1) eine veränderliche Höhe hat, um die Tiefe des Bohrens und/oder Präparierens des Zahnfleisches zu steuern, und wobei die Zone (Z2) eine konstante Höhe besitzt.

2. Vorrichtung nach Anspruch 1, **dadurch gekennzeichnet, dass** die zwei Bereiche (10a) und (10b) unterschiedliche Höhen haben.

3. Vorrichtung nach Anspruch 1, **dadurch gekennzeichnet, dass** die zwei Bereiche (10a) und (10b) die gleiche Höhe haben.

4. Vorrichtung nach Anspruch 1, **dadurch gekennzeichnet, dass** die Höhe der Zone (Z1) im Bereich von 3 bis 15 mm liegt und die konstante Höhe der Zone (Z2) etwa 5 mm beträgt.

5. Vorrichtung nach einem der Ansprüche 1 bis 4, **dadurch gekennzeichnet, dass** die Indexierungsmittel (11-12) aus äußeren Nasen, Gleitbahnen oder Ansätzen bestehen, die in eine äquivalente Anzahl komplementärer Formen des Typs Rinnen oder Kerben oder Umfangsnuten, die an dem Ring (13) gebildet sind, einrasten.

6. Vorrichtung nach Anspruch 5, **dadurch gekennzeichnet, dass** die äußeren Nasen, Gleitbahnen oder Ansätze diametral entgegengesetzt sind.

7. Vorrichtung nach einem der Ansprüche 1 bis 6, **dadurch gekennzeichnet, dass** sie aus chirurgischem Stahl, Titan oder einer Titanlegierung, aus PIC oder aus POM (Polyoxymethacrylat), aus Keramik oder aus Zirkon oder aus ihren Oxiden erhalten wird.

8. Vorrichtung nach einem der Ansprüche 1 bis 7, **dadurch gekennzeichnet, dass** die Biegung, die das Ende (10a1) des inneren Bereichs (10a) bildet, abgestumpft ist.

## Claims

1. A bone guiding and modeling device (UG) comprising a one-piece cylindrical body (10) provided with a bore for axially guiding a drill (15), and a ring (13), the lower end of said one-piece cylindrical body (10) being tapered to push back tissues towards the outside, the one-piece cylindrical body (10) comprising two adjacent concentric cylindrical seats (10a-10b), a first so-called inner seat (10b) for the passing and the guiding of the axis (15a) of the drilling tool (15) and a second so-called outer seat (10a) comprising at least two indexing means (11-12) adjusting in complementary shapes (13a) formed on said ring (13) capable of being rigidly attached to a surgical guide (14), **characterized in that** the indexing means (11-12) enable to block the guiding device depth-wise and rotation-wise on positioning of the one-piece cylindrical body (10) in the ring (13) rigidly attached to the surgical guide, and said inner seat (10b) having an outer diameter identical to the inner diameter of the outer seat (10a) and an inner diameter different from the inner diameter of the outer seat (10a), so that the outer cylindrical seat (10a) defines two successive areas (Z1-Z2) along its height, area (Z1) being the free portion coming out of the cylindrical body (10) after positioning thereof on the ring (13), area (Z2) being the inner portion located inside of the ring (13) after positioning of the cylindrical body (10) in the ring (13), said area (Z1) having a variable height to control the depth of the gum drilling and/or preparation, and area (Z2) having a constant height.

2. The device of claim 1, **characterized in that** the two seats (10a) and (10b) have different heights.

3. The device of claim 1 **characterized in that** the two seats (10a) and (10b) have the same height.

4. The device of claim 1, **characterized in that** the height of area (Z1) is set between 3 and 15 mm and the constant height of area (Z2) is set to approximately 5 mm.

5. The device of any of claims 1 to 4, **characterized in that** the indexing means (11-12) are external lugs, slides, or pegs fitting into an equivalent number of complementary shapes such as peripheral slots or notches or grooves formed on the ring (13).

6. The device of claim 5, **characterized in that** the external lungs, slides, or pegs are diametrically opposite.

7. The device of any of claims 1 to 6, **characterized in that** it is made of surgical steel, titanium, or a titanium alloy, of P.I.C. or of P.O.M. (PolyOxyMethacrylate), of ceramic, or of zirconia and of their oxides.

8. The device of any of claims 1 to 7, **characterized in that** the taper forming the end (10a1) of the inner seat (10a) is blunted.
